# EUROPÄISCHE PATENTSCHRIFT

(11) **EP 2 536 327 B1**
(45) Veröffentlichungstag und Bekanntmachung des Hinweises auf die Patenterteilung: **26.03.2014**
(21) Anmeldenummer: 10717518.4
(22) Anmeldetag: 15.02.2010
(51) Int. Cl.: A61B 3/113, A61F 9/008

(54) **VERFAHREN ZUM ERMITTELN VON ABWEICHUNGEN ZWISCHEN KOORDINATENSYSTEMEN VERSCHIEDENER TECHNISCHER SYSTEME**
METHOD FOR DETERMINING DEVIATIONS BETWEEN COORDINATE SYSTEMS OF VARIOUS TECHNICAL SYSTEMS
PROCEDE DE DETERMINATION D'ECARTS ENTRE DES SYSTEMES DE COORDONNEES DE DIFFERENTS SYSTEMES TECHNIQUES

(43) Veröffentlichungstag der Anmeldung: 26.12.2012
(73) Patentinhaber: WaveLight GmbH, 91058 Erlangen (DE)
(72) Erfinder: ABRAHAM, Mario, 90559 Burgthann (DE); MATSCHNIGG, Joachim, 90429 Nürnberg (DE); AGETHEN, Johannes, 91056 Erlangen (DE); KLAFKE, Mario, 63814 Mainaschaff (DE)
(74) Vertreter: Katérle, Axel
(86) Internationale Anmeldenummer: PCT/EP2010/000920
(87) Internationale Veröffentlichungsnummer: WO 2011/098098

(56) Entgegenhaltungen:
- WO-A1-01/44962
- WO-A1-2007/027562
- WO-A2-02/056789
- WO-A2-02/064031
- US-A- 4 134 681
- US-A1- 2003 223 037
- HONG YING YANG: PROC. OF SPIE, Bd. 7849, 10. Juli 2009 (2009-07-10), Seiten 748908-1-748908-4, XP040502516 online

## Beschreibung

Die Erfindung betrifft ein Verfahren zum Ermitteln von Abweichungen zwischen Koordinatensystemen verschiedener technischer Systeme.

Technische Systeme arbeiten häufig mit Koordinatensystemen, die sie zur Positionsbestimmung von aufgetretenen Ereignissen, von durchzuführenden Aktionen oder/und von ruhenden oder bewegten Objekten benötigen. Sensorsysteme, die den Ort eines Ereignisses oder eines Objekts in einem ein- oder mehrdimensionalen Koordinatensystem erfassen, sind ein Beispiel hierfür. Solche Sensorsysteme können beispielsweise kamerabasiert sein und als Bewegungsverfolgungssystem ausgestaltet sein. Derartige Bewegungsverfolgungssysteme werden eingesetzt, um die (veränderliche) Position sich bewegender Objekte zu erkennen und zu verfolgen.

Ein im Rahmen der Erfindung besonders betrachtetes Beispiel eines Bewegungsverfolgungssystems ist ein sogenannter Eye-Tracker, mit dem Augenbewegungen erfasst werden können. Der Einsatz von Eye-Trackern ist gängige Praxis in der laserchirurgischen Ophthalmologie, also der Bearbeitung des humanen Auges mittels Laserstrahlung zum Zwecke der Beseitigung oder zumindest Minderung von Fehlfunktionen oder krankhaften Erscheinungen des Auges.

Da das menschliche Auge - ohne mechanische Fixierung - niemals vollständig still steht, sondern selbst bei Anvisieren eines bestimmten Fixationsziels fortlaufend kleinere und größere Bewegungen ausführt (z.B. Sakkaden), wird bei verschiedenen Techniken der laserchirurgischen Augenbehandlung ein Eye-Tracker eingesetzt, um die Bewegungen des zu behandelnden Auges zu erfassen und abhängig von der erfassten Augenposition den Behandlungslaser zu steuern. Als diesbezügliches Beispiel ist insbesondere die refraktive Laserbehandlung zu nennen, bei der mittels Laserstrahlung im UV-Wellenlängenbereich korneales Gewebe ablatiert (d.h. von der Oberfläche abgetragen) wird, um auf diese Weise die Korneavorderfläche neu zu formen und hierdurch die Refraktionseigenschaften der Kornea zu verändern. Ein Beispiel einer solchen refraktiven Technik ist die sogenannte LASIK (Laser In Situ Keratomileusis), bei der zunächst mittels eines mechanischen Mikrokeratoms oder mittels Femtosekunden-Laserstrahlung ein in der Fachwelt üblicherweise als Flap bezeichnetes oberflächliches Deckelscheibchen aus der Kornea herausgeschnitten wird. Der Flap wird dabei nicht vollständig von der Kornea getrennt, sondern hängt noch in einem Scharnierbereich am übrigen Korneagewebe. Der Flap wird sodann zur Seite geklappt und das so freigelegte Korneamaterial einer ablatierenden Laserbehandlung unterzogen. Anschließend wird der Flap wieder zurückgeklappt. Weil die äußere Epithelschicht der Kornea bei dieser Methode nur wenig geschädigt wird, erfolgt die Wundheilung vergleichsweise schnell.

Lasereinrichtungen, die einen positionssteuerbaren Laserstrahl zur Bearbeitung eines Materials erzeugen, sind ein weiteres Beispiel für technische Systeme, die mit einem Koordinatensystem arbeiten. Die Ablationspositionen des Laserstrahls, also diejenigen Positionen, auf die der Laserstrahl gerichtet werden soll, können dabei durch Koordinatenpositionen in dem Koordinatensystem der Lasereinrichtung definiert werden. Bei Lasereinrichtungen, die einen gepulsten Laserstrahl erzeugen, kann jede Koordinatenposition einem einzelnen Laserpuls oder einer Gruppe von Laserpulsen zugeordnet sein.

Der obige Hinweis auf die Verwendung eines Eye-Trackers bei der laserchirurgischen Behandlung des humanen Auges verdeutlicht bereits, dass in der Praxis häufig Lösungen auftreten, bei denen mehrere technische Systeme mit jeweils eigenem Koordinatensystem zusammenarbeiten. Wenn eines der technischen Systeme Koordinatenwerte, die es mit Bezug auf sein eigenes Koordinatensystem ermittelt oder festgelegt hat, an ein anderes technisches System übergibt, das diese übergebenen Koordinatenwerte hernimmt, um beispielsweise die Koordinatenposition einer durchführenden Aktion in seinem Koordinatensystem zu bestimmten, können Probleme auftauchen, falls die Koordinatensysteme der beiden technischen Systeme nicht gegenseitig abgeglichen sind. Es ist ohne weiteres vorstellbar, dass ein bestimmter Punkt im Raum im Koordinatensystem des einen technischen Systems andere Koordinatenwerte haben kann als derselbe Raumpunkt im Koordinatensystem des anderen technischen Systems. Dies kann beispielsweise an einer unterschiedlichen Lage des Koordinatenzentrums der beiden Koordinatensysteme im Raum liegen. Es kann auch mit einer relativen Verdrehung der beiden Koordinatensysteme zueinander zu tun haben. Eine weitere Ursache kann in einer unterschiedlichen Skalierung der Koordinatenachsen liegen, d.h. derselbe nominelle Koordinatenwert entlang einer Achse kann bei einem Koordinatensystem in anderer Entfernung vom Koordinatenursprung als im anderen Koordinatensystem liegen.

Soweit die Koordinatensysteme verschiedener miteinander zusammenarbeitender technischer Systeme im Raum nicht zueinander identisch sind, ist es für ein einwandfreies Funktionieren der Zusammenarbeit zumindest notwendig, genaue Kenntnis über die unterschiedliche Raumlage oder/und die unterschiedliche Skalierung der Koordinatensysteme zu haben, um so eine Koordinatenposition aus einem der Koordinatensysteme in eine entsprechende Koordinatenposition eines anderen Koordinatensystems korrekt umrechnen zu können. Diese Kenntnis ist oft a priori nicht vorhanden und muss mühsam ermittelt werden.

US 4,134,681 befasst sich mit der Ermittlung der relativen Orientierung zweier physikalischer Systeme. Dabei wird für zwei Strahlvektoren, die beispielsweise jeweils die Richtung eines Laserstrahls angeben, die jeweilige Vektorrichtung in den Koordinatensystemen der beiden physikalischen Systeme ermittelt, so dass insgesamt vier Richtungsvektoren erhalten werden, zwei für jedes Koordinatensystem. Aus diesen vier Richtungsvektoren wird sodann die relative Orientierung der beiden Koordinatensysteme und somit der beiden physikalischen Systeme ermittelt.

Aufgabe der Erfindung ist es, eine einfache und vor allem der automatisierten Durchführung zugängliche Methode zum Ermitteln von Abweichungen zwischen Koordinatensystemen verschiedener technischer Systeme anzugeben.

Zur Lösung dieser Aufgabe sieht die Erfindung in Übereinstimmung mit den Merkmalen des Anspruchs 1 ein Verfahren zum Ermitteln von Abweichungen zwischen Koordinatensystemen verschiedener technischer Systeme vor, umfassend
- Ermitteln einer Koordinatenposition eines Referenzmerkmals eines Testobjekts im Koordinatensystem eines ersten der technischen Systeme,
- Anbringen mindestens eines Testmerkmals an dem Testobjekt, wobei das Testmerkmal im Koordinatensystem eines zweiten der technischen Systeme an einer Koordinatenposition angebracht wird, die abhängig von der ermittelten Koordinatenposition des Referenzmerkmals festgelegt wird,
- Ermitteln einer Koordinatenposition des mindestens einen Testmerkmals oder/und mindestens eines hiervon abgeleiteten Merkmals im Koordinatensystem des ersten technischen Systems, und

Ermitteln von Abweichungen zwischen den Koordinatensystemen des ersten und des zweiten technischen Systems zumindest auf der Basis von:
(a) der ermittelten Koordinatenposition des mindestens einen Testmerkmals oder/und des mindestens einen hiervon abgeleiteten Merkmals im Koordinatensystem des ersten technischen Systems und
(b) der Koordinatenposition des Referenzmerkmals im Koordinatensystem des ersten technischen Systems.

Bei der erfindungsgemäßen Lösung kommt in einer Ausgestaltung ein Testobjekt zum Einsatz, das mit einem Referenzmuster versehen ist, welches von einem ersten der technischen Systeme detektierbar ist. Das Referenzmuster kann unmittelbar das Referenzmerkmal bilden. Alternativ kann das Referenzmuster so ausgestaltet sein, dass sich aus ihm ein Referenzmerkmal eindeutig herleiten lässt. Beispielsweise kann das Referenzmerkmal das Zentrum (Mittelpunkt) eines als Referenzmuster dienenden geometrischen Objekts bilden. Algorithmen, die aus einer detektierten geometrischen Form den Mittelpunkt errechnen, sind an sich bekannt und brauchen an dieser Stelle nicht im Detail erläutert zu werden. In jedem Fall ist in einer bevorzugten Ausgestaltung das erste technische System in der Lage, auf Basis des detektierten Referenzmusters die Koordinatenposition des Referenzmerkmals in seinem Koordinatensystem (d.h. im Koordinatensystem des ersten technischen Systems) zu ermitteln.

Die so ermittelte Koordinatenposition (dargestellt durch einen oder mehrere Koordinatenwerte) wird sodann von dem ersten technischen System an ein zweites der technischen Systeme übergeben. Das zweite technische System verwendet die übergebenen Koordinatenwerte des Referenzmerkmals, als seien es Koordinatenwerte seines eigenen Koordinatensystems (d.h. des Koordinatensystems des zweiten technischen Systems), und ermittelt in seinem Koordinatensystem die Koordinatenposition für ein zu erzeugendes Testmerkmal nach Maßgabe einer vorgegebenen Erzeugungsregel abhängig von der übergebenen Koordinatenposition des Referenzmerkmals. Beispielsweise kann für das Testmerkmal die Erzeugungsregel vorgegeben sein, dass es längs zumindest eines Teils der Koordinatenachsen des Koordinatensystems des zweiten technischen Systems jeweils einen vorgegebenen Koordinatenabstand von dem Referenzmerkmal haben soll. Durch eine solche Erzeugungsregel wird in dem Koordinatensystem des zweiten technischen Systems die Position des Testmerkmals mit Bezug auf die Position des Referenzmerkmals eindeutig festgelegt.

In einer bevorzugten Ausgestaltung bringt das zweite technische System sodann das Testmerkmal an der in vorstehender Weise abhängig von der Koordinatenposition des Referenzmerkmals ermittelten Koordinatenposition an. Sofern mehrere Testmerkmale anzubringen sind, geht das zweite technische System in entsprechender Weise für jedes der Testmerkmale vor.

In einem anschließenden Schritt wird das Testobjekt mit dem Referenzmuster und den angebrachten Testmerkmalen wieder von dem ersten technischen System untersucht. Dabei ermittelt das erste technische System, welche Koordinatenposition das mindestens eine Testmerkmal oder/und mindestens ein hiervon abgeleitetes Merkmal im Koordinatensystem des ersten technischen Systems hat. Basierend hierauf können nun eine oder mehrere Abweichungen innerhalb des Koordinatensystems des ersten technischen Systems ermittelt werden. Vorzugsweise wird zumindest ein Verschiebungsvektor ermittelt, um den das Koordinatensystem des zweiten technischen Systems im Raum gegenüber dem Koordinatensystem des ersten technischen Systems verschoben ist, oder/und es wird eine relative Verdrehung zwischen den Koordinatensystemen der beiden technischen Systeme ermittelt oder/und es werden Skalierungsunterschiede zwischen den Koordinatensystemen der beiden technischen Systeme ermittelt.

Die ermittelten Abweichungen können in einen oder mehrere Korrekturfaktoren umgerechnet werden, die von dem zweiten technischen System im späteren Betrieb zur Korrektur etwaiger Koordinatenpositionen herangezogen werden, die es von dem ersten technischen System übergeben bekommt. Auf diese Weise gelingt ein Abgleich der Koordinatensysteme der beiden technischen Systeme.

In einer bevorzugten Ausgestaltung werden im Rahmen des erfindungsgemäßen Verfahrens mehrere Testmerkmale an unterschiedlichen Stellen des Testobjekts angebracht. Zumindest ein Teil der Testmerkmale kann dabei in einer Polygonanordnung, beispielsweise einer Viereckanordnung, um das Referenzmerkmal herum an dem Testobjekt angebracht werden. Als ein abgeleitetes Merkmal kann dann eine Polygonmitte der polygonal angeordneten Testmerkmale ermittelt werden und es kann im Koordinatensystem des ersten technischen Systems eine Abweichung zwischen der Koordinatenposition des Referenzmerkmals und der Koordinatenposition der Polygonmitte ermittelt werden.

Gemäß einer Weiterbildung werden die Abweichungen zwischen den Koordinatensystemen des ersten und des zweiten technischen Systems ferner auf der Basis einer Soll-Koordinatenposition wenigstens eines Merkmals unter den Test- und abgeleiteten Merkmalen im Koordinatensystem des ersten technischen Systems ermittelt. Die Soll-Koordinatenposition eines Testmerkmals kann beispielsweise durch Anwendung der zuvor erwähnten Erzeugungsregel im Koordinatensystem des ersten technischen Systems ermittelt werden. Wenn beispielsweise die Erzeugungsregel für das Testmerkmal einen vorbestimmten x-Abstand und einen vorbestimmten y-Abstand von dem Referenzmerkmal längs zweier Achsen x, y des Koordinatensystems des zweiten technischen Systems vorsieht, so kann die Soll-Koordinatenposition des Testmerkmals im Koordinatensystem des ersten technischen Systems dadurch ermittelt werden, dass dieselben nominellen (zahlenmäßigen) Koordinatenabstände auf die ermittelte Koordinatenposition des Referenzmerkmals angewendet werden.

Gemäß einer bevorzugten Ausführungsform umfasst das erste technische System eine Bewegungsverfolgungseinrichtung mit einer auf das Testobjekt gerichteten Kamera, wobei die Bewegungsverfolgungseinrichtung die Koordinatenposition des Referenzmerkmals und des mindestens einen Test- oder/und abgeleiteten Merkmals in einem ersten Koordinatensystem ermittelt.

Das Testobjekt kann ein Muster tragen, dessen Zentrum die Bewegungsverfolgungseinrichtung als Referenzmerkmal ermittelt. Das Muster (Referenzmuster) kann beispielsweise ein flächiges Muster sein, das gegenüber dem umgebenden Bereich optisch kontrastiert. Der optische Kontrast sollte zumindest an der Grenze des Referenzmusters zum Umgebungsbereich bestehen. Er kann zumindest teilweise durch unterschiedliche Graustufen oder unterschiedliche Farbtöne des Musters und des umgebenden Bereichs bewirkt sein. Alternativ oder zusätzlich ist es möglich, den Kontrast zwischen dem Referenzmuster und dem umgebenden Bereich zu erzeugen oder zu verstärken, indem beide Bereiche unterschiedlich strukturiert werden oder einer der Bereiche strukturiert wird und der andere Bereich strukturlos bleibt. Beispielsweise kann der Umgebungsbereich des Referenzmusters mit einem Netz aus aufgedruckten Punkten oder Linien versehen sein, während das Referenzmuster strukturlos ist und allein mit einem bestimmten Grau- bzw. Farbton vollflächig ausgefüllt ist.

Es ist im übrigen nicht notwendig, dass das Referenzmuster oder/und der Umgebungsbereich jeweils nur eine einzige Farbe besitzen. Es kann ohne weiteres ein Farb- oder Graustufenverlauf innerhalb des Referenzmusters oder/und innerhalb des Umgebungsbereichs realisiert werden.

Das Referenzmuster kann einen runden Umriss besitzen, beispielsweise einen kreisrunden oder elliptischen Umriss. Auf dies Weise kann eine zweidimensionale Projektion einer humanen Pupille simuliert werden. Dabei kann die Größe des Referenzmusters zumindest näherungsweise einer humanen Pupille entsprechen. Das Referenzmuster stellt in diesem Fall eine Pupillennachbildung dar. Dies ist insofern zweckmäßig, als Bildverarbeitungsalgorithmen, die aus einer bildlich erfassten Pupille eines Auges die Position des Pupillenzentrums berechnen, an sich bekannt sind und marktgängig erhältlich sind. Ein Testobjekt mit einer solchen Pupillennachbildung eignet sich deshalb besonders für die Anwendung der Erfindung im Rahmen einer Vorrichtung für die laserchirurgische Behandlung des humanen Auges. Es versteht sich freilich, dass nichtrunde Umrissformen des Referenzmusters gleichermaßen möglich sind, soweit sichergestellt ist, dass das Referenzmuster ein eindeutig bestimmbares Zentrum besitzt. Auch muss das Referenzmuster nicht größenmäßig einer humanen Pupille entsprechen. Es kann größer oder kleiner sein.

Das zweite technische System umfasst bevorzugt eine Laseranordnung, welche das mindestens eine Testmerkmal mittels eines Laserstrahls, insbesondere eines gepulsten Laserstrahls, an dem Testobjekt anbringt. Dabei nutzt die Laseranordnung ein zweites Koordinatensystem zur Positionierung des Laserstrahls.

Um mit einem Eye-Tracker oder allgemein einer kamerabasierten Bewegungsverfolgungseinrichtung das angebrachte Testmerkmal gut erkennen zu können, wird empfohlen, dass zur Anbringung eines Testmerkmals das Testobjekt derart mit dem Laserstrahl behandelt wird, dass eine lokale Verfärbung oder/und eine lokale Kraterbildung des Testobjekts entsteht.

Die ermittelte mindestens eine Abweichung wird zweckmäßigerweise zur Korrektur von Koordinatendaten verwendet, die das zweite technische System von dem ersten technischen System übergeben bekommt und die es für seinen Betrieb benötigt. Die ermittelte mindestens eine Abweichung kann hierzu in einen oder mehrere geeignete Korrektur- oder Kalibrierfaktoren umgerechnet werden, die auf die vom ersten technischen System übergebenen Koordinatendaten angewendet werden.

Das Testobjekt besitzt vorzugsweise ein sich optisch abhebendes Muster (Referenzmuster) und ist zumindest in einem Bereich um das Muster derart ausgestaltet, dass durch lokale Lasereinstrahlung sich optisch abhebende Testmerkmale erzeugbar sind.

Bevorzugt ist das Muster ein flächenhaftes Muster, das einer humanen Pupille nachgebildet sein kann und in einer ersten Farbe erscheint, während das Testobjekt in einem Bereich um das Muster in einer zweiten Farbe erscheint. Die zweite Farbe ist dabei von der ersten Farbe verschieden. Der Begriff Farbe soll hier breit verstanden werden. Unterschiedliche Farben können beispielsweise durch unterschiedliche Farbtöne (einschließlich Grau) oder durch unterschiedliche Graustufen oder durch unterschiedliche Hellwerte eines Farbtons realisiert sein.

Gemäß einem Beispiel kann die erste Farbe auf ein Substrat des Testobjekts aufgedruckt sein. Im Umgebungsbereich um das Referenzmuster kann das Substrat eine einzige Farbschicht mit einer von der ersten Farbe verschiedenen Farbe besitzen. Bei Lasereinstrahlung kann sich diese Farbschicht (z.B. Weiß) dann verfärben und so die Testmerkmale erkennbar werden lassen. Es ist aber auch möglich, dass das Substrat in dem Umgebungsbereich übereinander mehrere unterschiedliche Farbschichten aufweist, von denen die oberste (äußerste) die zweite Farbe zeigt, so dass das Testobjekt in dem Umgebungsbereich in der zweiten Farbe erscheint. Bei Lasereinstrahlung kann dann die zweite Farbe ausbleichen oder anderweitig verschwinden, wodurch die darunter liegende Farbschicht lokal zum Vorschein kommt. Dies gewährleistet einerseits die gute Erkennbarkeit des Referenzmusters und andererseits die gute Erkennbarkeit des mindestens einen Testmusters.

Das Testobjekt ist beispielsweise platten- oder blattförmig ausgebildet. Beispielsweise kann es ein Stück Papier oder Pappe aufweisen, das auf einer seiner Flachseiten das Muster trägt und dort zugleich zur Erzeugung der Testmerkmale ausgestaltet ist. Das Papier- oder Pappstück kann beispielsweise auf eine stabile Tragplatte aus Metall oder Kunststoff aufgeklebt sein, um das Testobjekt insgesamt ausreichend steif und robust zu machen.

In einer Abwandlung kann das Testobjekt eine gekrümmte (oder allgemein dreidimensionale) Oberfläche besitzen, auf der das Referenzmuster angebracht ist und die Testmerkmale angebracht werden können. Beispielsweise kann diese Oberfläche der Vorderfläche eines humanen Auges nachgebildet sein. Für den Abgleich der Koordinatensysteme kann es dann erforderlich sein, zusätzlich die Krümmung bzw. den Krümmungsverlauf der Oberfläche des Testobjekts zu berücksichtigen, um etwaige Skalierungsfehler zu vermeiden.

Schließlich betrifft die Erfindung noch eine Vorrichtung für die laserchirurgische Ophthalmologie, umfassend
- eine Laseranordnung zur Bereitstellung eines gepulsten fokussierten Laserstrahls und zum Richten desselben auf ein zu behandelndes Auge,
- einen Eye-Tracker zur Erfassung von Bewegungen des Auges,
- eine mit dem Eye-Tracker gekoppelte Steuereinheit, welche dazu eingerichtet ist, die Laseranordnung abhängig von der erfassten Bewegung des Auges zu steuern, wobei die Steuereinheit ferner dazu eingerichtet ist:
   (i) Die Durchführung eines Verfahrens der vorstehend beschriebenen Art zu bewirken, um Abweichungen zwischen einem von dem Eye-Tracker verwendeten ersten Koordinatensystem und einem von der Laseranordnung verwendeten zweiten Koordinatensystem zu ermitteln, und
   (ii) ermittelte Abweichungen bei der Steuerung der Laseranordnung zu berücksichtigen.

Die Erfindung wird nachfolgend anhand der beigefügten Zeichnungen weiter erläutert. Es stellen dar:
Figur 1 in stark schematisierter Blockdarstellung ein Ausführungsbeispiel einer Vorrichtung für die laserchirurgische Ophthalmologie,
Figur 2 ein Beispiel für eine voneinander abweichende räumliche Lage zweier in der Vorrichtung der Figur 1 verwendeter Koordinatensysteme und
Figur 3 ein Ausführungsbeispiel eines zur Kalibrierung der Vorrichtung der Figur 1 verwendbaren Testobjekts.

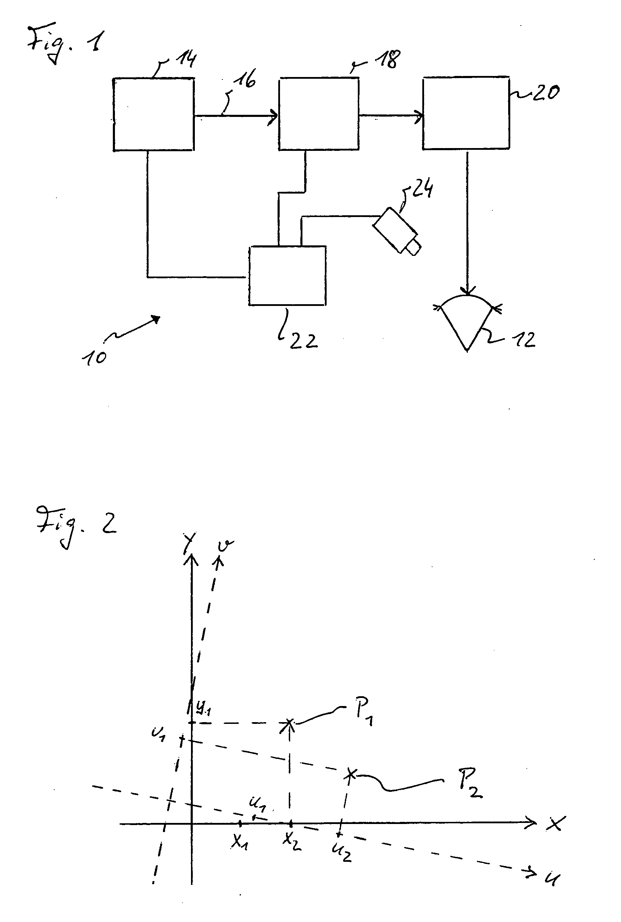

Die in Figur 1 gezeigte, allgemein mit 10 bezeichnete Vorrichtung für die laserchirurgische Ophthalmologie ist beispielsweise für die Durchführung kornealer Ablationen eines zu behandelnden Auges 12 eingerichtet. Sie umfasst eine Laserquelle 14, welche einen gepulsten Laserstrahl 16 erzeugt, der mittels einer steuerbaren Ablenkeinheit (Scanner) 18 gezielt in einer zur Strahlrichtung normalen Ebene (im weiteren Verlauf x-y-Ebene genannt) ablenkbar ist. Dem Scanner 18 nachgeschaltet ist eine Fokussiereinheit 20, welche den Laserstrahl 16 auf das zu behandelnde Auge 12 fokussiert.

Für ablatierende Behandlungen liegt die Wellenlänge der von der Laserquelle 14 erzeugten Laserstrahlung im UV-Bereich. Beispielsweise umfasst die Laserquelle 14 einen bei 193nm strahlenden Excimer-Laser.

Der Scanner 18 ist beispielsweise in an sich bekannter Weise von einem Paar galvanometrisch steuerbarer Ablenkspiegel gebildet. Die Fokussiereinheit 20 kann von einer Einzellinse oder von einem mehrlinsigen Linsensystem gebildet sein.

Eine elektronische Steuereinheit 22 steuert die Laserquelle 14 und den Scanner 18 nach Maßgabe eines durch ein Steuerprogramm implementierten, für den Patienten zuvor ermittelten Ablationsprofils. Das Ablationsprofil gibt an, wie viel Korneamaterial an welcher Stelle des zu behandelnden Auges abgetragen werden muss. Jeder Laserpuls (Schuss) bewirkt einen bestimmten Materialabtrag. Das Steuerprogramm bewirkt, dass an jeder Stelle des Ablationsbereichs so viele Laserpulse gesetzt werden, wie benötigt werden, um die jeweils gewünschte Materialhöhe abzutragen. Die Ablationspositionen sind durch Koordinatenpaare dargestellt, die sich auf die beiden Achsen eines die erwähnte x-y-Ebene aufspannenden (rechtwinkligen) x-y-Koordinatensystems beziehen. Die Koordinatenpaare geben dabei den Koordinatenabstand von einem Zentrum des Ablationsprofils (Ablationszentrum) an, das typischerweise abhängig vom Pupillenzentrum des zu behandelnden Auges 12 definiert ist. Unvermeidliche Bewegungen des Auges 12 führen dazu, dass sich die Lage des Pupillenzentrums und folglich des Ablationszentrums in dem x-y-Koordinatensystem fortlaufend ändert.

Zur Überwachung der Augenbewegungen umfasst die Vorrichtung 10 eine kurz als Eye-Tracker bezeichnete Augenverfolgungseinrichtung 24, welche mit einer Kamera beispielsweise ein Infrarotbild des Auges 12 und insbesondere der Iris mitsamt der Pupille aufnimmt und mittels geeigneter Bildverarbeitungssoftware die Lage des Pupillenzentrums berechnet. Der Eye-Tracker 24 arbeitet jedoch nicht mit demselben x-y-Koordinatensystem, mit dem die Steuereinheit 22 und der Scanner 18 arbeiten. Stattdessen berechnet sie die Lage des Pupillenzentrums in einem eigenen (rechtwinkligen) Koordinatensystem, das nachfolgend zum Zwecke der Veranschaulichung als u-v-Koordinatensystem bezeichnet wird. In diesem u-v-Koordinatensystem gibt der Eye-Tracker 24 die Position des Pupillenzentrums gleichermaßen durch ein Koordinatenpaar an, also jeweils einen Koordinatenwert für die u-Achse und die v-Achse.

Die Steuereinheit 22 erhält von dem Eye-Tracker 24 die u-v-Koordinatenposition des Pupillenzentrums und rechnet diese in die entsprechende x-y-Koordinatenposition um. Hierbei greift sie auf vorab ermittelte Korrekturinformationen zurück, die etwaige räumliche Lageabweichungen und etwaige Skalierungsabweichungen zwischen den beiden Koordinatensystemen repräsentieren. Die Korrekturinformationen können beispielsweise in Form einer Korrekturfunktion oder in Form eines oder mehrer multiplikativ oder additiv zu verwendender Korrekturfaktoren implementiert sein. Aus der in x-y-Koordinaten umgerechneten Position des Pupillenzentrums kann die Steuereinheit 22 sodann die aktuelle Lage des Ablationszentrums berechnen und den Scanner 18 entsprechend steuern.

Figur 2 zeigt schematisch mögliche Abweichungen zwischen dem von der Steuereinheit 22 und dem Scanner 18 verwendeten x-y-Koordinatensystem und dem von dem Eye-Tracker 24 benutzten u-v-Koordinatensystem. Zur besseren Unterscheidbarkeit ist das x-y-Koordinatensystem mit durchgezogenen Linien gezeichnet, während das u-v-Koordinatensystem mit gestrichelten Linien gezeichnet ist.

Als eine erste Abweichung der beiden Koordinatensysteme im Raum erkennt man im Beispielfall der Figur 2 eine unterschiedliche Lage der Koordinatenursprünge, also der Kreuzungspunkte der Koordinatenachsen. Diese unterschiedliche Raumlage kann durch einen Verschiebungsvektor ausgedrückt werden.

Als eine zweite Abweichung erkennt man im Beispielfall der Figur 2 eine relative Verdrehung der beiden Koordinatensysteme. Das Maß dieser Verdrehung kann durch einen Winkelwert angegeben werden.

Eine dritte Abweichung der Koordinatensysteme kann in einer unterschiedlichen Skalierung bestehen. Zur Veranschaulichung dieses Sachverhalts sind in Figur 2 auf der x-Achse zwei Koordinatenwerte x₁, x₂ kenntlich gemacht, die nominell zwei auf der u-Achse eingezeichneten Koordinatenwerten u₁, u₂ entsprechen. Nominelle Entsprechung heißt, dass die Koordinatenwerte x₁ und u₁ zahlenmäßig gleich sind und die Koordinatenwerte x₂ und u₂ ebenfalls zahlenmäßig gleich sind.

Allerdings ist leicht erkennbar, dass der Koordinatenwert x₁ einen deutlich geringeren Abstand vom Zentrum des x-y-Koordinatensystems hat als der Koordinatenwert u₁ vom Zentrum des u-v-Koordinatensystems. Gleichermaßen ist der Abstand des Koordinatenwerts x₂ vom Zentrum des x-y-Koordinatensystems kleiner als der Abstand, den der Koordinatenwert u₂ vom Zentrum des u-v-Koordinatensystems hat. Bei übereinstimmenden Zahlenwerten von x₁ und u₁ und übereinstimmenden Zahlenwerten von x₂ und u₂ bedeutet dies, dass die Skalierung der x-Achse eine andere ist als die Skalierung der u-Achse.

In ähnlicher Weise ist in Figur 2 auf der y-Achse ein Koordinatenwert y₁ eingezeichnet, der hinsichtlich seines Zahlenwerts einem auf der v-Achse eingezeichneten Koordinatenwert v₁ entspricht. Allerdings sind auch hier die Abstände der Koordinatenwerte y₁, v₁ vom Zentrum des jeweiligen Koordinatensystems unterschiedlich. Es ist nämlich der Abstand des Koordinatenwerts y₁ vom Zentrum des x-y-Koordinatensystems erheblich größer als der Abstand des Koordinatenwerts v₁ vom Zentrum des u-v-Koordinatensystems. Dies bedeutet auch eine unterschiedliche Skalierung der y-Achse im Vergleich zur Skalierung der v-Achse.

Solche Skalierungsabweichungen können für alle Achsen der Koordinatensysteme oder auch nur für einen Teil der Achsen bestehen.

Jede der erläuterten drei möglichen Abweichungen führt dazu, dass Punkte, die im x-y-Koordinatensystem und im u-v-Koordinatensystem durch dieselben Koordinatenwerte beschrieben sind, unterschiedliche Lage haben. Veranschaulicht ist dies in Figur 2 durch zwei Beispielpunkte P₁ und P₂. Der Punkt P₁ ist durch die Koordinatenwerte x₂, y₁ definiert, während der Punkt P₂ durch die Koordinatenwerte u₂, v₁ definiert ist. Trotz gleicher Zahlenwerte für x₂ und u₂ sowie für y₁ und v₁ ergibt sich dennoch ein deutlicher Lageabstand der Punkte P₁, P₂. Ohne die erwähnten Abweichungen (Zentrumsverschiebung, Verdrehung, Skalierungsunterschied) würden die Punkte P₁, P₂ dagegen zusammenfallen.

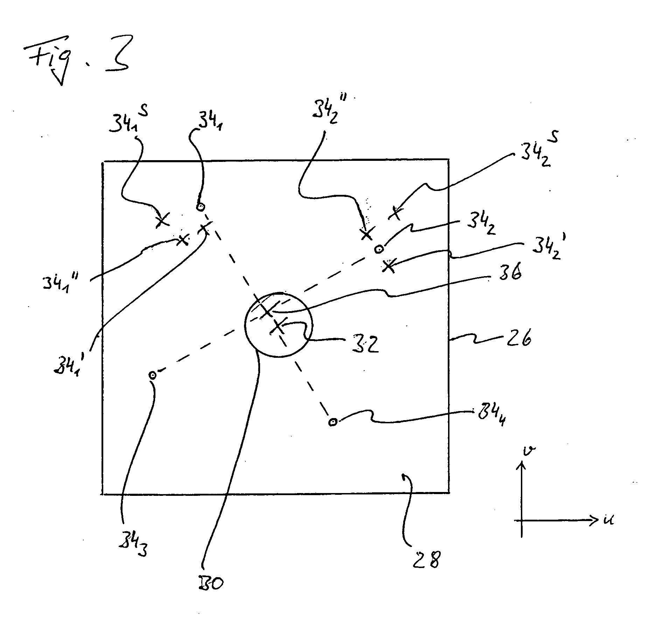

Anhand der Figur 3 wird nun ein Ausführungsbeispiel eines Verfahrens erläutert, um Abweichungen zwischen zwei Koordinatensystemen verschiedener technischer Systeme zu ermitteln. Im konkreten Beispielfall wird hierbei auf die von der Steuereinheit 22 und dem Scanner 18 einerseits und dem Eye-Tracker 24 andererseits verwendeten Koordinatensysteme Bezug genommen.

Zunächst wird mittels des Eye-Trackers 24 ein Testobjekt 26 untersucht, das zweckmäßigerweise im wesentlichen an derjenigen Position in die Vorrichtung 10 eingelegt wird, an der sich später das zu behandelnde Auge 12 befindet. Insbesondere wird das Testobjekt 26 so angeordnet, dass es sich in der Fokusebene des Laserstrahls 16 befindet.

Das Testobjekt 26 besitzt im gezeigten Beispielfall eine ebene Oberseite 28, welche näherungsweise mittig ein sich optisch vom umgebenden Bereich abhebendes Referenzmuster 30 trägt. Das Referenzmuster 30 ist einer Pupille nachgebildet und ist dementsprechend von einem näherungsweise pupillengroßen, vorzugsweise farblich voll ausgefüllten Kreismuster gebildet. Das Kreismuster 30 muss nicht zwangsläufig exakt kreisrund sein; es kann auch mehr oder weniger starke Abweichungen von der Kreisform zeigen.

Die in dem Eye-Tracker 24 enthaltene Bildverarbeitungssoftware erkennt das Pupillenmuster 30 und berechnet hieraus die Lage des Zentrums des Musters in seinem u-v-Koordinatensystem. Das Zentrum des Musters ist in Figur 3 bei 32 angedeutet; es stellt ein Referenzmerkmal im Sinne der Erfindung dar.

Der Eye-Tracker 24 übergibt die u-v-Koordinaten des Kreiszentrums 32 an die Steuereinheit 22. Diese steuert daraufhin die Laserquelle 14 und den Scanner 18, um durch Lasereinstrahlung eine Mehrzahl von Testmerkmalen 34 auf der Oberseite 28 des Testobjekts 26 anzubringen. Die Testmerkmale 34 sind beispielsweise kleine Kreise oder andere geometrische Formen, die sich optisch von den umliegenden Bereichen der Oberseite 28 des Testobjekts 26 abheben und von dem Eye-Tracker 24 detektierbar sind. Beispielsweise kann die Erzeugung jedes der Testmerkmale 34 die Einstrahlung mehrerer hundert oder sogar mehrerer tausend Laserpulse benötigen.

Die Positionen, an denen die Testmerkmale 34 angebracht werden, werden von der Steuereinheit 22 abhängig von der vom Eye-Tracker 24 gemeldeten u,v-Koordinatenposition des Kreiszentrums 32 berechnet. Eine vorgegebene Erzeugungsregel legt fest, an welchen x,y-Positionen die Testmerkmale 34 mit Bezug auf das Kreiszentrum 32 anzubringen sind. Eine beispielhafte Erzeugungsregel kann vorgeben, dass vier Testmerkmale 34₁ ... 34₄ in einer Viereckanordnung um das Kreiszentrum 32 herum anzubringen sind, wobei das Kreiszentrum 32 die Viereckmitte bilden soll. Eine solche Viereckanordnung von vier Testmerkmalen 34 ist in Figur 3 beispielhaft gezeigt. Die Viereckanordnung ist dort angenähert eine Quadratanordnung.

Im gezeigten Beispielfall der Fig. 3 ist zu erkennen, dass die angebrachten Test-merkmale 34 tatsächlich nicht zentrisch zu dem Kreiszentrum 32 liegen, sondern ein gegenüber dem Kreiszentrum 32 versetztes, als Kreuzungspunkt zweier Quadratdiagonalen definiertes Quadratzentrum 36 haben. Der Versatz zwischen dem Kreiszentrum 32 und dem Quadratzentrum 36 lässt auf Abweichungen zwischen dem u-v-Koordinatensystem des Eye-Trackers 24 und dem x-y-Koordinatensystem der Steuereinheit 22 und des Scanners 18 schließen. Denn im Fall solcher Abweichungen können zwei Punkte mit denselben zahlenmäßigen Koordinatenwerten im u-v-Koordinatensystem und im x-y-Koordinatensystem auseinanderfallen, wie in Fig. 2 anhand der Punkte P₁ und P₂ veranschaulicht. Ein Punkt im x-y-Koordinatensystem mit denselben Koordinatenwerten wie das Kreiszentrum 32 liegt deshalb räumlich nicht deckungsgleich zu dem Kreiszentrum 32, sondern versetzt hierzu. Da die Testmerkmale 34 mit Bezug auf diesen (versetzten) Punkt im x-y-Koordinatensystem erzeugt werden, liegen sie zentrisch zu diesem Punkt, nicht aber zentrisch zu dem Kreiszentrum 32.

Zur quantitativen Erfassung der Abweichungen zwischen den beiden Koordinatensystemen wird das mit den Testmerkmalen 34 versehene Testobjekt 26 nun erneut von dem Eye-Tracker 24 abgetastet, um die u,v-Koordinaten der Testmerkmale 34 im u-v-Koordinatensystem zu ermitteln. Aus den u,v-Koordinaten der Testmerkmale 34 werden zudem die u,v-Koordinaten des Quadratzentrums 36 berechnet. Das Quadratzentrum 36 stellt ein abgeleitetes Merkmal im Sinne der Erfindung dar, denn es ist aus den Testmerkmalen 34 abgeleitet.

Auf Basis der so ermittelten u,v-Positionen der Testmerkmale 34 und des Quadratzentrums 36 werden von der Steuereinheit 22 Informationen ermittelt, welche die Abweichungen zwischen dem u-v-Koordinatensystem und dem x-y-Koordinatensystem charakterisieren.

Konkret erlauben der u-Abstand und der v-Abstand des Quadratzentrums 36 vom Kreiszentrum 32 die Festlegung eines Verschiebungsvektors, der Stärke und Richtung einer Abweichung der Lage der Ursprünge der Koordinatensysteme charakterisiert. Gemäß einem Beispiel berechnet die Steuereinheit dann für mindestens ein Paar der Testmerkmale 34 zugeordnete erstkorrigierte u,v-Positionen, welche gegenüber dem betreffenden Testmerkmal 34 um den Verschiebungsvektor verschoben sind. Die erstkorrigierten u,v-Positionen sind dementsprechend gegenüber dem Kreiszentrum 32 zentriert. Beispielhaft sind in Fig. 3 für die Testmerkmale 34₁, 34₂ solche erstkorrigierten u,v-Positionen 34₁', 34₂' eingezeichnet, die gegenüber dem Testmerkmal 34₁ bzw. 34₂ jeweils um dasselbe Maß verschoben sind, um welches das Quadratzentrum 36 gegenüber dem Kreiszentrum 32 verschoben ist.

Die Ermittlung einer relativen Verdrehung der beiden Koordinatensysteme kann beispielsweise dadurch erfolgen, dass die Steuereinheit 22 für dasselbe Paar der Testmerkmale, für das sie die erstkorrigierten u,v-Positionen ermittelt hat, Soll-Koordinatenpositionen im u-v-Koordinatensystem ermittelt. Hierzu wendet sie die erwähnte Erzeugungsregel für die Testmerkmale im u-v-Koordinatensystem mit Bezug auf die u,v-Koordinatenposition des Kreiszentrums 32 an. Beispielhaft sind in Fig. 3 die so ermittelte Soll-Position des Testmerkmals 34₁ bei 34₁^{S} und die Soll-Position des Testmerkmals 34₂ bei 34₂^{S} im u-v-Koordinatensystem eingezeichnet.

Eine Verdrehung der Koordinatensysteme ist einfach dadurch feststellbar, dass die Verbindungsgerade der Soll-Koordinatenpositionen 34₁^{S} und 34₂^{S} mit der Verbindungsgerade der erstkorrigierten u,v-Positionen 34₁' und 34₂' verglichen wird. Liegen diese beiden Verbindungsgeraden parallel, sind die Koordinatensysteme nicht verdreht. Liegen sie winkelig zueinander, gibt der Winkel zwischen den Verbindungsgeraden den Verdrehwinkel der Koordinatensysteme an.

Um etwaige Skalierungsunterschiede zwischen den beiden Koordinatensystemen zu ermitteln, kann die Steuereinheit 22 anhand des ermittelten Verdrehwinkels der Koordinatensysteme aus den erstkorrigierten u,v-Positionen des betreffenden Testmerkmalpaars zugeordnete zweitkorrigierte u,v-Positionen ermitteln, welche zusätzlich zu dem Verschiebungsmaß um den Verdrehwinkel korrigiert sind. Beispielhaft sind in Fig. 3 zweitkorrigierte u,v-Positionen 34₁", 34₂" für die Testmerkmale 34₁, 34₂ eingezeichnet. Die Verbindungsgerade dieser zweitkorrigierten u,v-Positionen 34₁", 34₂" liegt nun parallel zur Verbindungsgerade der Soll-Koordinatenpositionen 34₁^{S}, 34₂^{S}. Allerdings liegen die zweitkorrigierten u,v-Positionen 34₁", 34₂" weiterhin nicht deckungsgleich mit den Soll-Koordinatenpositionen 34₁^{S}, 34₂^{S}. Dies ist ein Hinweis auf eine unterschiedliche Achsenskalierung der Koordinatensysteme.

Durch Berechnung des u-Abstands der Soll-Koordinatenpositionen 34₁^{S}, 34₂^{S} und des u-Abstands der zweitkorrigierten u,v-Positionen 34₁", 34₂" und durch Vergleich (insbesondere Quotientenbildung) dieser u-Abstände kann eine etwaige unterschiedliche Skalierung der u-Achse des u-v-Koordinatensystems und der x-Achse des x-y-Koordinatensystems erkannt und quantitativ ermittelt werden. Das gleiche gilt für eine etwaige unterschiedliche Skalierung der v-Achse und der y-Achse, die durch Berechnung des v-Abstands der Soll-Koordinatenpositionen 34₁^{S}, 34₂^{S} und des v-Abstands der zweitkorrigierten u,v-Positionen 34₁", 34₂" und durch Vergleich (insbesondere Quotientenbildung) dieser v-Abstände erkannt und quantitativ ermittelt werden kann.

Statt des u-Abstands bzw. des v-Abstands der Soll-Koordinatenpositionen und der zweitkorrigierten u,v-Positionen eines Testmerkmalspaars kann zur Ermittlung einer abweichenden Achsenskalierung der Koordinatensysteme alternativ auch der u-Abstand oder/und der v-Abstand zwischen der Soll-Koordinatenposition eines Testmerkmals und dem Kreiszentrum 32 und zwischen der zweitkorrigierten u,v-Position desselben Testmerkmals und dem Kreiszentrum 32 ermittelt werden.

Es versteht sich, dass eine Viereckanordnung der Testmerkmale 34 rein beispielhaft ist und dass andere polygonale Anordnungen und sogar auch eine Kreisanordnung der Testmerkmale 34 ohne weiteres möglich sind.

Um die Testmerkmale 34 optisch erkennbar zu machen, kann der Bereich der Testobjektoberseite 28 um das Referenzmuster 30 herum mit einer Farbe bedruckt sein, die bei Lasereinstrahlung verschwindet und dadurch eine darunterliegende andere Farbe zum Vorschein kommen lässt. Das Testobjekt 26 kann zu diesem Zweck ein plattenförmiges oder blattförmiges Substrat umfassen, das auf einer seiner Flachseiten mit einer Untergrundfarbe flächig bedruckt ist. Auf diese Untergrundfarbe ist im Bereich des Referenzmusters 30 eine erste andere Farbe aufgedruckt, die das Referenzmuster 30 bildet. Außerhalb des Referenzmusters 30 ist eine durch die Laserstrahlung ausbleichbare oder anderweitig entfernbare zweite andere Farbe aufgedruckt.

In einer alternativen Ausgestaltung ist es vorstellbar, im Bereich außerhalb des Referenzmusters 30 ein Gitternetz von eng nebeneinanderliegenden, feinen Linien aufzudrucken. Durch lokale Einstrahlung von Laserstrahlung kann dabei das Gitternetz an den bestrahlten Stellen unterbrochen werden, beispielsweise indem eine durch Laserstrahlung ausbleichbare Farbe für das Gitternetz verwendet wird oder indem mit der Laserstrahlung ein Krater in der Oberseite 28 des Testobjekts 26 erzeugt wird. Die so erzeugte Unterbrechung im Gitternetz kann mittels geeigneter Bildverarbeitungssoftware erkannt und als Testmerkmal genutzt werden.

Die Vorrichtung 10 kann das oben erläuterte Verfahren zur Ermittlung von Abweichungen zwischen den u-v- und x-y-Koordinatensystemen vollautomatisch durchführen, sobald von einem Benutzer das Testobjekt 26 eingelegt worden ist und ein entsprechender Startbefehl gegeben wird. Insbesondere kann die Steuereinheit 22 als Teil einer derartigen automatischen Kalibrierung geeignete Korrekturparameter für die Koordinatentransformation aus dem u-v-Koordinatensystem in das x-y-Koordinatensystem ermitteln und in einem Speicher (nicht näher dargestellt) abspeichern.

## Patentansprüche

1. Verfahren zum Ermitteln von Abweichungen zwischen Koordinatensystemen verschiedener technischer Systeme, umfassend
- Ermitteln einer Koordinatenposition eines Referenzmerkmals (32) eines Testobjekts (26) im Koordinatensystem (u,v) eines ersten (24) der technischen Systeme,
- Anbringen mindestens eines Testmerkmals (34) an dem Testobjekt, wobei das Testmerkmal im Koordinatensystem (x,y) eines zweiten (18, 22) der technischen Systeme an einer Koordinatenposition angebracht wird, die abhängig von der ermittelten Koordinatenposition des Referenzmerkmals festgelegt wird,
- Ermitteln einer Koordinatenposition des mindestens einen Testmerkmals oder/und mindestens eines hiervon abgeleiteten Merkmals (36) im Koordinatensystem (u,v) des ersten technischen Systems, und
- Ermitteln von Abweichungen zwischen den Koordinatensystemen des ersten und des zweiten technischen Systems zumindest auf der Basis von:
(a) der ermittelten Koordinatenposition des mindestens einen Testmerkmals oder/und des mindestens einen hiervon abgeleiteten Merkmals im Koordinatensystem (u,v) des ersten technischen Systems und
(b) der Koordinatenposition des Referenzmerkmals im Koordinatensystem (u,v) des ersten technischen Systems, wobei die Abweichungen zwischen den Koordinatensystemen des ersten und des zweiten technischen Systems ferner auf der Basis einer Soll-Koordinatenposition wenigstens eines Merkmals unter den Test- und abgeleiteten Merkmalen im Koordinatensystem (u,v) des ersten technischen Systems ermittelt werden.

2. Verfahren nach Anspruch 1, wobei mehrere Testmerkmale (34) an unterschiedlichen Stellen des Testobjekts (26) angebracht werden.

3. Verfahren nach Anspruch 2, wobei zumindest ein Teil der Testmerkmale (34) in einer Polygonanordnung, beispielsweise einer Viereckanordnung, um das Referenzmerkmal herum an dem Testobjekt angebracht wird.

4. Verfahren nach Anspruch 3, wobei als ein abgeleitetes Merkmal eine Polygonmitte (36) der polygonal angeordneten Testmerkmale ermittelt wird und im Koordinatensystem (u,v) des ersten technischen Systems eine Abweichung zwischen der Koordinatenposition des Referenzmerkmals (32) und der Koordinatenposition der Polygonmitte ermittelt wird.

5. Verfahren nach einem der vorhergehenden Ansprüche, wobei die Soll-Koordinatenposition eines Testmerkmals (34) im Koordinatensystem (u,v) des ersten technischen Systems nach Maßgabe eines für dieses Merkmal vorgegebenen Koordinatenabstands von dem Referenzmerkmal (32) im Koordinatensystem (x,y) des zweiten technischen Systems ermittelt wird.

6. Verfahren nach einem der vorhergehenden Ansprüche, wobei das erste technische System eine Bewegungsverfolgungseinrichtung (24) mit einer auf das Testobjekt gerichteten Kamera umfasst, wobei die Bewegungsverfolgungseinrichtung die Koordinatenposition des Referenzmerkmals (32) und des mindestens einen Test- oder/und abgeleiteten Merkmals (34, 36) in einem ersten Koordinatensystem (u,v) ermittelt.

7. Verfahren nach Anspruch 6, wobei das Testobjekt (26) ein Muster (30) trägt, dessen Zentrum die Bewegungsverfolgungseinrichtung als Referenzmerkmal (32) ermittelt.

8. Verfahren nach Anspruch 7, wobei das Muster (30) ein flächiges Muster ist, das gegenüber dem umgebenden Bereich optisch kontrastiert.

9. Verfahren nach Anspruch 8, wobei das Muster (30) einen runden Umriss besitzt, insbesondere einen kreisrunden oder elliptischen Umriss.

10. Verfahren nach einem der Ansprüche 6 bis 9, wobei das zweite technische System eine Laseranordnung umfasst, welche das mindestens eine Testmerkmal (34) mittels eines Laserstrahls (16), insbesondere eines gepulsten Laserstrahls, an dem Testobjekt (26) anbringt, wobei die Laseranordnung ein zweites Koordinatensystem (x,y) zur Positionierung des Laserstrahls nutzt.

11. Verfahren nach Anspruch 10, wobei zur Anbringung eines Testmerkmals (34) das Testobjekt (26) derart mit dem Laserstrahl (16) behandelt wird, dass eine lokale Verfärbung oder/und eine lokale Kraterbildung des Testobjekts entsteht.

12. Verfahren nach einem der vorhergehenden Ansprüche, wobei die ermittelte mindestens eine Abweichung zur Korrektur von Koordinatendaten verwendet wird, die das zweite technische System von dem ersten technischen System übergeben bekommt.

13. Verfahren nach einem der vorhergehenden Ansprüche, wobei das Testobjekt (26) ein sich optisch abhebendes Muster (30) besitzt und zumindest in einem Bereich um das Muster derart ausgestaltet ist, dass durch lokale Lasereinstrahlung sich optisch abhebende Testmerkmale erzeugbar sind.

14. Verfahren nach Anspruch 13, wobei das Muster (30) ein flächenhaftes, vorzugsweise einer humanen Pupille nachgebildetes Muster ist, welches in einer ersten Farbe erscheint, wobei das Testobjekt in einem Bereich um das Muster in einer zweiten Farbe erscheint.

15. Verfahren nach Anspruch 13 oder 14, wobei das Testobjekt (26) plattenoder blattförmig ausgebildet ist.

16. Vorrichtung für die laserchirurgische Ophthalmologie, umfassend
- eine Laseranordnung (14, 18, 20) zur Bereitstellung eines gepulsten fokussierten Laserstrahls und zum Richten desselben auf ein zu behandelndes Auge (12),
- einen Eye-Tracker (24) zur Erfassung von Bewegungen des Auges,
- eine mit dem Eye-Tracker gekoppelte Steuereinheit (22), welche dazu eingerichtet ist, die Laseranordnung abhängig von erfassten Bewegungen des Auges zu steuern, wobei die Steuereinheit ferner dazu eingerichtet ist:
(i) die Durchführung des Verfahrens nach einem der Ansprüche 1 bis 15 zu bewirken, um Abweichungen zwischen einem von dem Eye-Tracker verwendeten ersten Koordinatensystem (u,v) und einem von der Laseranordnung verwendeten zweiten Koordinatensystem (x,y) zu ermitteln, und
(ii) ermittelte Abweichungen bei der Steuerung der Laseranordnung zu berücksichtigen.

## Claims

1. Method for determining deviations between coordinate systems of different technical systems, comprising
- determination of a coordinate position of a reference feature (32) of a test object (26) in the coordinate system (u,v) of a first (24) of the technical systems,
- creation of at least one test feature (34) on the test object, with the test feature being created in the coordinate system (x,y) of a second (18, 22) of the technical systems at a coordinate position that is determined in dependence on the determined coordinate position of the reference feature,
- determination of a coordinate position of the at least one test feature and/or at least one feature (36) derived from it in the coordinate system (u,v) of the first technical system
- determination of deviations between the coordinate systems of the first and second technical systems, at least on the basis of:
(a) the determined coordinate position of the at least one test feature and/or of the at least one feature derived from it in the coordinate system (u,v) of the first technical system and
(b) the coordinate position of the reference feature in the coordinate system (u,v) of the first technical system,
wherein the deviations between the coordinate systems of the first and the second technical system are further determined on the basis of a target coordinate position of at last one feature among the test and derived features in the coordinate system (u, v) of the first technical system.

2. Method according to claim 1, wherein multiple test features (34) are created at various locations of the test object (26).

3. Method according to claim 2, wherein at least a part of the test features (34) are created in a polygonal array, for example a rectangular array, around the reference feature on the test object.

4. Method according to claim 3, wherein as a derived feature a polygonal center (36) of the test features arrayed as a polygon is determined and in the coordinate system (u, v) of the first technical system a deviation is determined between the coordinate position of the reference feature (32) and the coordinate position of the polygon center.

5. Method according to one of the foregoing claims, wherein the target coordinate position of a test feature (34) is determined in the coordinate system (u, v) of the first technical system in accordance with a coordinate distance preset for this feature from the reference feature (32) in the coordinate system (x, y) of the second technical system.

6. Method according to one of the foregoing claims, wherein the first technical system comprises a motion tracking device (24) with a camera directed toward the test object, wherein the motion tracking system determines the coordinate position of the reference feature (32) and of the at least one test and/or derived feature (34, 36) in a first coordinate system (u, v).

7. Method according to claim 6, wherein the test object (26) is provided with a pattern (30), the center of which is determined by the motion tracking device as a reference feature (32).

8. Method according to claim 7, wherein the pattern (30) is an areal pattern that contrasts optically vis-à-vis the surrounding area.

9. Method according to claim 8, wherein the pattern (30) possesses a round contour, especially a circular or elliptical contour.

10. Method according to one of claims 6 to 9, wherein the second technical system comprises a laser device, which creates the at least one test feature (34) by means of a laser beam (16), especially a pulsed laser beam, on the test object (26), wherein the laser device uses a second coordinate system (x, y) for positioning of the laser beam.

11. Method according to claim 10, wherein for creation of a test feature (34), the test object (26) is treated by the laser beam (16) so that the test object undergoes a local color change and/or a local cratering.

12. Method according to one of the foregoing claims, wherein the at least one deviation determined is used for correction of coordinate data, which the second technical system receives, transmitted from the first technical system.

13. Method according to one of the foregoing claims, wherein the test object (26) possesses a pattern (30) that stands out optically and at least in an area around the pattern is configured so that through local laser irradiation, test features that stand out optically can be generated.

14. Method according to claim 13, wherein the pattern (30) is an areal pattern, preferably modeling a human pupil, which appears in a first color, whereas the test object appears in an area around the pattern in a second color.

15. Method according to claim 13 or 14, wherein the test object (26) is configured to be plate- or sheet-shaped.

16. Device for laser-surgical ophthalmology, comprising
- a laser device (14, 18, 20) for providing a pulsed focused laser beam and directing same toward an eye (12) to be treated,
- an eye-tracker (24) for detecting eye movements,
- a control unit coupled (22) with the eye-tracker, which is configured to control the laser device in dependence on detected eye movements, wherein the control unit is additionally configured to:
(i) cause the method according to one of claims 1 to 15 to be carried out, to determine deviations between a first coordinate system (u, v) used by the eye-tracker and a second coordinate system (x, y) used by the laser device, and
(ii) to take into account determined deviations when controlling the laser device.

## Revendications

1. Procédé de détermination d'écarts entre les systèmes de coordonnées des différents systèmes techniques, comportant pour étapes
- la détermination d'une position de coordonnées d'une caractéristique de référence (32) d'un objet à mesurer (26) dans le système de coordonnées (u, v) d'un premier système technique (24),
- l'application d'au moins une caractéristique de test (34) à l'objet à mesurer, cette caractéristique de test étant appliquée à une position de coordonnées dans le système de coordonnées (x, y) d'un deuxième système technique (18, 22), laquelle position est définie en fonction de la position de coordonnées déterminée de la caractéristique de référence,
- la détermination d'une position de coordonnées de ladite caractéristique de test ou/et d'au moins une caractéristique (36) qui en est dérivée dans le système de coordonnées (u, v) du premier système technique, et
- la détermination d'écarts entre les systèmes de coordonnées des premier et deuxième systèmes techniques au moins sur la base de :
(a) la position de coordonnées déterminée de ladite caractéristique de test ou/et d'au moins une caractéristique (36) qui en est dérivée dans le système de coordonnées (u, v) du premier système technique
(b) la position de coordonnées de la caractéristique de référence dans le système de coordonnées (u, v) du premier système technique, les écarts entre les systèmes de coordonnées des premier et deuxième systèmes techniques étant en outre déterminés sur la base d'une position de coordonnées théorique d'au moins une caractéristique parmi les caractéristiques de test et les caractéristiques dérivées dans le système de coordonnées (u, v) du premier système technique.

2. Procédé selon la revendication 1, dans le cadre duquel plusieurs caractéristiques de test (34) sont appliquées à différents endroits de l'objet à mesurer (26).

3. Procédé selon la revendication 2, dans le cadre duquel au moins une partie des caractéristiques de test (34) est appliquée à l'objet à mesurer dans une configuration polygonale, par exemple dans une configuration carrée, autour de la caractéristique de référence.

4. Procédé selon la revendication 3, dans le cadre duquel il est déterminé, comme caractéristique dérivée, un centre de polygone (36) des caractéristiques de test disposées de manière polygonale, ainsi qu'un écart entre la position de coordonnées de la caractéristique de référence (32) et la position de coordonnées dudit centre de polygone, dans le système de coordonnées (u, v) du premier système technique.

5. Procédé selon l'une des revendications précédentes, dans le cadre duquel la position de coordonnées théorique d'une caractéristique de test (34) dans le système de coordonnées (u, v) d'un premier système technique est déterminée selon une distance de coordonnées prédéfinie pour cette caractéristique de la caractéristique de référence (32) dans le système de coordonnées (x, y) du deuxième système technique.

6. Procédé selon l'une des revendications précédentes, dans le cadre duquel le premier système technique englobe un dispositif de suivi de mouvement (24) pourvu d'une caméra dirigée sur l'objet à mesurer, ce dispositif de suivi de mouvement déterminant la position de coordonnées de la caractéristique de référence (32) et de ladite caractéristique de test ou/et de ladite caractéristique dérivée (34, 36) dans un premier système de coordonnées (u, v).

7. Procédé selon la revendication 6, dans le cadre duquel l'objet à mesurer (26) porte un motif (30) dont le centre est déterminé comme caractéristique de référence (32) par le dispositif de suivi de mouvement.

8. Procédé selon la revendication 7, dans le cadre duquel ledit motif (30) est un motif plat qui contraste optiquement avec la zone environnante.

9. Procédé selon la revendication 8, dans le cadre duquel le motif (30) possède un contour rond, plus particulièrement un contour circulaire ou elliptique.

10. Procédé selon l'une des revendications 6 à 9, dans le cadre duquel le deuxième système technique englobe un agencement laser lequel applique ladite caractéristique de test (34) au moyen d'un rayon laser (16), plus particulièrement au moyen d'un rayon laser pulsé, sur l'objet à mesurer (26), ledit agencement laser utilisant un deuxième système de coordonnées (x, y) pour le positionnement du rayon laser.

11. Procédé selon la revendication 10, dans le cadre duquel l'objet à mesurer (26) est traité au rayon laser (16) en vue de l'application d'une caractéristique de test (34) de telle sorte qu'il se produit une coloration locale ou/et qu'il se forme localement un cratère sur l'objet à mesurer.

12. Procédé selon l'une des revendications précédentes, dans le cadre duquel ledit écart déterminé est utilisé pour la rectification des données de coordonnées que le deuxième système technique reçoit du premier système technique.

13. Procédé selon l'une des revendications précédentes, dans le cadre duquel l'objet à mesurer (26) possède un motif (30) qui se détache optiquement, et est réalisé au moins dans une zone entourant le motif de telle sorte que des caractéristiques de test qui se détachent optiquement peuvent être générées par rayonnement laser.

14. Procédé selon la revendication 13, dans le cadre duquel le motif (30) est un motif plat, de préférence un motif calqué sur une pupille humaine, lequel motif apparaît dans une première couleur, l'objet à mesurer apparaissant dans une deuxième couleur dans une zone entourant le motif.

15. Procédé selon la revendication 13 ou la revendication 14, dans le cadre duquel l'objet à mesurer (26) est réalisé sous la forme d'une plaque ou d'une feuille.

16. Dispositif pour la chirurgie de l'oeil au laser, comprenant
- un agencement de laser (14, 18, 20) pour la fourniture d'un rayon laser pulsé focalisé et pour l'orientation de celui-ci sur un oeil à traiter (12),
- un oculomètre (24) pour détecter les mouvements de l'oeil,
- une unité de commande (22) couplée audit oculomètre (24), laquelle est aménagée pour commander l'agencement laser en fonction des mouvements oculaires détectés, cette unité de commande étant aménagée pour :
(i) mettre en oeuvre le procédé selon l'une des revendications 1 à 15 afin de déterminer des écarts entre un premier système de coordonnées (u, v) utilisé par l'oculomètre et un deuxième système de coordonnées (x, y) utilisé par l'agencement laser, et
(ii) prendre en compte les écarts déterminés lors de la commande de l'agencement laser.
